# EUROPEAN PATENT SPECIFICATION

(11) **EP 0 934 526 B1**
(45) Date of publication and mention of the grant of the patent: **08.01.2003**
(21) Application number: 97943221.8
(22) Date of filing: 07.10.1997
(51) Int. Cl.: G01N 33/554, G01N 33/68

(54) **METHODS AND MEANS FOR SELECTING PEPTIDES AND PROTEINS HAVING SPECIFIC AFFINITY FOR A TARGET**
VERFAHREN UND MITTEL ZUR AUSWAHL VON PEPTIDEN UND PROTEINEN MIT SPEZIFISCHER AFFINITÄT ZU EINEM ZIELMOLEKÜL
PROCEDES ET SYSTEMES PERMETTANT DE SELECTIONNER DES PEPTIDES ET DES PROTEINES QUI POSSEDENT UNE AFFINITE SPECIFIQUE ENVERS UNE CIBLE

(30) Priority: 08.10.1996 EP 96202791
(43) Date of publication of application: 11.08.1999
(73) Proprietor: U-BiSys B.V., 3584 CX Utrecht (NL)
(72) Inventor: LOGTENBERG, Ton, NL-3433 CH Vreeswijk (NL); DE KRUIF, Cornelis, Adriaan, NL-3514 AN Utrecht (NL)
(74) Representative: Ottevangers, Sietse Ulbe
(86) International application number: NL9700557
(87) International publication number: WO98015833

(56) References cited:
- WO-A-92/08738
- WO-A-92/15677
- WO-A-95/15982
- WO-A-95/31723
- DE KRUIF ET AL.: "Selection and application of human single chain Fv antibody ...." JOURNAL OF MOLECULAR BIOLOGY, vol. 248, no. 1, 21 April 1995, LONDON, pages 97-105, XP000196590 cited in the application
- GEYSEN ET AL: "USE OF PEPTIDE SYNTHESIS TO PROBE VIRAL ANTIGENS FOR EPITOPES TO A RESOLUTION OF A SINGLE AMINO ACID" PROCEEDINGS OF THE NATIONAL ACADEMY OF SCIENCES OF USA, vol. 81, July 1984, WASHINGTON DC, pages 3998-4002, XP000609021 cited in the application

## Description

This invention relates to the field of proteinaceous biological molecules having specific affinity for a target. More specifically the invention relates to such molecules derived from specific binding peptides such as proteins such as antibodies, in particular monoclonal antibodies or specific binding derivatives or fragments thereof. In an exemplary embodiment the invention relates to peptides and antibodies which are more or less randomly produced as large collections of different molecules (libraries) expressed on the surface of a replicable genetic package. From these libraries peptides or antibodies are affinity-selected for binding to the target molecule.

Specific binding affinities between biological molecules or derivatives thereof have been used throughout applied biotechnology, molecular biology, medical biology, etc. for a very long period of time for many different purposes. Some of the most commonly applied biological molecules having a specific binding affinity are antibodies. Antibodies are complex proteins usually composed of two light chains and two heavy chains which comprise constant regions and variable regions which variable regions again comprise hypervariable domains, which determine the binding specificity of the antibody (complementarity determining regions, (CDR)). Antibodies are part of the normal defense system of higher vertebrates. They are produced by cells of the immune system. Until 1975 antibodies were obtained by administrating an antigenic substance to an animal (often a rodent) and harvesting the antibodies produced by the animal in its immune response. This resulted in mixtures of antibodies having many different specifities for different binding sites on the antigenic substance. These mixtures are referred to as polyclonal antisera.

The advent of monoclonal antibodies (Kohler and Milstein (1975) Nature 256:495) represented a significant technical breakthrough with important consequences both scientifically and commercially. Monoclonal antibodies are conventionally made by fusing antibody-secreting plasma cells from an immunized animal with a tumor cell line in order to immortalize the former plasma cells. The immortalized hybridomas are screened for the production of antibodies with the desired specificities. Selected hybridomas are expanded to large numbers and the monoclonal antibodies secreted into the medium are harvested and purified for application.
Since the arrival of hybridoma technology, monoclonal antibodies have found broad application in procedures aimed at examining and identifying many antigens in many formats from pregnancy tests to AIDS tests. They are also widely applied in research laboratories for use in studying and isolating a broad variety of molecules and for immunohistochemistry, i.e. identifying certain cells in tissues. For instance, monoclonal antibodies that are reactive with specific antigens expressed on subpopulations of cells can be utilized to detect these cells in tissue sections using immunohistochemical or immunofluorescent approaches. The inherent advantages of monoclonal compared to polyclonal antibodies is that they react only with one specific epitope and that they are standardized from batch to batch and from laboratory to laboratory.

Despite of all the advantages obtainable using monoclonal antibodies, there are a number of limitations associated with hybridoma technology and therefore with the monoclonal antibodies that can be obtained. For instance, the number of hybridomas that can be established and screened for the secretion of a certain antibody specificity is limited. The immunization protocol and the manner in which the animals immune system handles the antigen favors the selection of hybridomas scereting antibodies against immunodominant epitopes, whereas other specificities will not be obtained because the immunizing antigen or some of its epitopes are not recognized by the animals immune system. In addition, the MoAbs obtained from animals are hardly suitable for therapeutical use in humans because they evoke an immune respons. Some of these drawbacks may be circumvented by using antibody engineering, aiming at chimeric antibodies of which the variable regions are of animal origin and the constant regions are of human origin (the constant region is responsible for the bulk of the immune response). However these chimeras still evoke an immune response. Another possibility to avoid the immune response is a technique called CDR-grafting, whereby only the CDR's are of animal origin, which CDR's are inserted in a human antibody framework. These humanized antibodies are rather difficult to make and often loose some of their binding affinity.

Another way of modifying antibodies is trying to obtain the smallest fragment that will still specifically bind the antigen (or to be more precise the epitope) with a significant affinity. The smaller the fragment, the less immunogenic it may be. This has resulted in Fab'2, Fab fragments, to peptide-like molecules comprising only one of the three CDR's present on each heavy and light chain of mammalian antibodies, i.e. CDR3 of the heavy chain.
In genetically engineering antibodies ways have also been found to avoid having to express at least two different subunits (a heavy and a light chain) for obtaining one antibody. This has been achieved by making a fusion through a peptide linker between a heavy and a light chain subunit. This results in so-called single chain antibodies, which will be discussed in more detail later on.

Apart from the drawbacks related to immunogenicity of monoclonal antibodies, there is also a limitation in the specificities obtainable with monoclonal antibodies. Nature employs a specific process in making CDR's of antibodies through a complex process of hypermutations, rearrangements and insertions in the gene fragment encoding the CDR, followed by a selection of antibody producing cells in primary and secondary lymphoid organs. The result of these processes is that the variety of binding specificities that can be produced in an animal is limited. As one result thereof monoclonal antibodies are not always useful in the application they are intended for, such as for example immunohistochemistry.

Immunohistochemical and immunofluorescent procedures involving tissue sections generally consist of the steps of embedding a tissue in an embedding medium, with or withour prior or subsequent fixation or freezing of the tissue, cutting the embedded tissue in thin sections, incubating the tissue sections with monoclonal antibodies, amplification of the signal and visualisation of cells that have bound antibody by an enzymatic reaction and precipitation of substrate or by fluorescent labels.
A drawback of conventional monoclonal antibodies is that they often do not bind to target epitopes in fixed tissue sections, presumably as a result of destruction of the target epitope by the fixation procedure. Fixation and embedding procedures such as paraffin embedding an formalin fixation, that are optimal for preservation of the anatomical structure of the tissue and are routinely used for medical diagnosis, lead to extensive protein denaturation thus limiting the application of monoclonal antibodies that often bind to native epitopes.
Another breakthrough in specific binding technology involves the use of replicable genetic display packages. The term replicable genetic display package or display package describes a biological particle which has genetic information providing the particle with the ability to replicate. The package can display a fusion protein including an antibody, antibo dy fragment, protein or peptide. The fusion protein is presented by the display pac kage in a context which permits the antibody to bind a target structure that is contac ted with the display package. The display package can be, for example, derived from bacterial cells, bacterial spores or bacterial virusses. In phage display technology a gene fusion is made between a sequence encoding a peptide of interest and a sequence encoding a protein displayed on the outside of a phage (actually the peptide sequence is inserted in the proteins encoding sequence). Libraries of very large numbers of display packages containing all kinds of different peptides within one of their surface proteins can be easily produced and selected and tested based on their binding affinity for other molecules or cells, etc.

Parts of human antibody genes and sequences encoding single chain variable regions have been inserted in such phage genes and thus anibody fragment phage display libraries have been obtained. Display libraries of antibody variable regions may be constructed by a variety of methods. In general, a large collection of antibodies or antibody-fragments is expressed on the surface of a replicable genetic display package. In an exemplary embodiment of the present invention, the display package is a phage particle which comprises an antibody-coat protein fusion; the nucleotide sequences used for construction of the antibodies are of human origin. The library of surface expressed antibody molecules is brought into contact with the target antigen for affinity selection of antibodies that bind to the antigen. This procedure allows the rapid screening of very large collections of displayed antibody molecules, yields human antibodies and does not require immunization of an animal. Novel specificities may be obtained from such libraries.

Phages expressing antibody specificities of interest are selected from libraries in a four step procedure 1) affinity binding of phages to the target antigen, 2) removal of non-bound phages by washing 3) elution of bound phages and 4) infection and propagation of eluted phages in E Coli bacteria. A variety of targets has been successfully employed to obtain phage antibodies from libraries by affinity selection. Phage antibody affinity selections have been performed on purified antigens coated to a solid phase, on intact eukaryotic cells in suspension or in monolayers and on prokaryotic cells.

The language "affinity binding" and "affinity selection" as well as "differential binding" refer to the separation of members of the peptide or antibody display library based on the differing abilities of these molecules on the surface of each of the display packages of the library to bind to the target structure. Examples of affinity selection include affinity chromatography, immunoprecipitation, fluorescence activated cell sorting, agglutination and plaque lifts.

In all phage antibody affinity selection procedures, it is imperative to obtain a large collection of binding phages in the first round of selection, preferably binding to diverse regions of the target. In subsequent rounds of selection, procedures may be implemented to select from this collection those antibodies that fulfill specific requirements.

The present invention provides further selection methods whereby novel specificities can be easily obtained and/or useful antibody fragments and epitopes (or even any combination of two peptides or proteinaceous substances having binding affinity for each other) can be identified and obtained.
In one embodiment the invention provides a method for identifying a peptide or antibody capable of specific binding to a proteinaceous target, comprising displaying the peptide or antibody on the surface of a replicable display package, synthesizing sets of oligopeptides derived from the proteinaceous target on a solid phase and contacting the specific binding peptide with the solid-phase-bound oligopeptide to allow for binding. The peptides expressed on the replicable display package may be linear or have another conformation as for example in di-sulfide bridged circular peptides or peptides that contain more than one disulfide bridge and have an unprodictable conformation. In addition, the target oligopeptides may be linear or comprise another configuration.

Thus the present invention provides a method that allows the directed pre-selection of phage antibodies (or other binding peptides) to defined sets of oligopeptides representing (sub)regions of the target molecule. For the purpose of the present invention, the term antibody in its various grammatical forms is art-recognized and includes immunoglobulin molecules and immunologically active portions and/or derivatives thereof, i.e. molecules that contain an antigen binding site. The peptides are synthesized in overlapping or non-overlapping sets of multimers on polystyrene rods or other solid phases such as membranes or beads. In the preferred embodiment, polystyrene rods used for pepscan technology are used. After selection on solid phase-bound rods, collections of phages enriched for anti-oligopeptide phage antibodies can be used in subsequent rounds to select anti-oligopeptide phages that also bind to the target molecule in a another configuration or to select antibodies that discriminate between various forms of the target proteinaceous substance. Many examples of anti-oligopeptide antibodies that bind to the target protein the oligopeptide is derived from have been described in the scientific literature. Herein oligopeptides are defined as comprising 6-30, preferably 8-20 amino acid residues. They will also be simply referred to as peptides.

The term pepscan relates to a procedure for rapid concurrent synthesis on solid supports of hundreds of peptides, essentially as described (Geysen H., et al., Proc. Natl. Acad. Sci. USA 81, 3998 (1984). In the original procedure, the peptides are synthesized on polyethylene rods as solid support. Other solid supports such a membraneous filters or beads may also be used. Various peptide formats may be synthesized including linear peptides and di-sulfide bridged circular peptides.

Thus in one embodiment the present invention provides a method for generating antibodies specific for sets of peptides synthesized on a solid phase such as the polyethylene rods used in pepscan technology. The subject method generally comprises the steps of synthesizing on a solid phase sets of peptides spanning a target molecule or a subregion thereof; the construction of a display library of antibodies or peptides; the selection of displayed peptides or antibodies that bind to the solid-phage-bound peptides using affinity selection and affinity absorption techniques.

In exemplary embodiments, the display library of peptides or antibodies can be a phage display library or a display library generated on bacterial or yeast cell surface.
After one or multiple rounds of selection, the binding displayed antibodies or peptides may be subjected to additional rounds of selection on peptides or on the target protein in its native or denatured configuration. In exemplary embodiments, the subject method can be used to isolate anti-peptide antibodies that also bind to the target protein in its native configuration, for example a marker expressed on normal or malignant eukaryotic cells, or in denatured configuration such as markers expressed on cells in fixed tissue sections. Likewise, the subject method can be used to generate antibodies which can discriminate between a protein and other related forms of the protein, by using a subtractive approach. The related forms of the protein can differ by one or more amino acids from the target protein or differ as a result of post translational modifications such as glycosylation.

In an illustrative embodiment of the subject method, a specific antibody can be generated by affinity selection using a synthetic antibody phage display library. The phage library is incubated with the pepscan block containing rods covered with peptides spanning the extracellular region of a transmembrane protein and phages are allowed to bind to individual rods. After removal of non-bound phages by washing, bound phages are eluted from the rods and propagated in bacterial cells. Multiple subsequent rounds of selection may be performed using the same approach. Alternatively, the binding phages from the first round of selection on solid phase-bound peptides may be selected for binding to the protein in another configuration such as that expressed on a cell or a recombinant protein. In the subtractive approach, phages eluted from the peptides in the first and/or subsequent round(s) of selection may be incubated with rods covered with other peptides, such as peptides differing one or more amino acids from the peptide used for the initial selection, in order to absorb phages that crossreact with related molecules.
It is contemplated that the present invention can be applied to obtain bi-specific antibodies that bind to two non-overlapping epitopes on the same molecule of mono meric antigen. Such antibodies have been shown to have enhanced affinity due to the chelate effect (Cheong, H.S., et al. Biochem. Biophys. Res. Comm 173, 795 (1990)). The DNA encoding the pool of phage antibodies affinity selected for binding to a set of overlapping peptides may be recloned into vectors permitting the display of various formats of bi-specific antibodies. The displayed bi-specific antibodies may be affinity selected for binding to the target protein to obtain high affinity binding phages. Uisng the same strategy, bi-specific antibodies may be obtained that bind to two epitopes that are expressed on different molecules.

It is also contemplated by the present invention that individual antibodies or peptides, and the genes or oligonucleotides encoding these antibodies or peptides, can be isolated from the phage display library after affinity enrichment. These genes and oligonucleotides can be used for subcloning into expression vectors for production of antibodies and derivatives of the oligonucleotides can be used for nucelotide se quencing and the information used to produce chemically synthesized peptides or peptides produced by recombinant DNA methods such as production in prokaryotic or eukaryotic expression systems.
In an alternative embodiment the present invention provides a method for identifying a peptide capable of specific binding to a fixated biological target, comprising displaying the peptide on the surface of a replicable display package and contacting said specific binding peptide with the fixated target to allow for binding.

Thus the present invention provides a method that facilitates the selection of antibodies that are suitable for immmunohistochemical and immunofluorescent procedures with tissue sections that have been embedded and fixed with a variety of procedures. In addition, the procedure facilitates the isolation of antibody specificities that bind to particular anatomical regions of a tissue section, for example a region infiltrated by tumor cells by using a subtraction procedure and/or micromanipulation of individual cells or small groups of cells. The method employs large libraries of antibodies or peptides expressed on the surface of a replicable display package. The display libraries may be constructed by a variety of methods. In an exemplary embodiment the display package is a phage particle which comprises an antibody-coat protein fusion; the nucleotide sequences used for construction of the antibodies are partially synthesized and assembled in vitro. These libraries contain many novel specificities, including antibodies against self antigens.

In the basic approach, phages expressing antibody specificities of interest are selected from this library in again a four step procedure 1) binding of phages to the embedded and fixed tissue sections, 2) removal of non-bound phages by washing 3) elution of bound phages and 4) infection and propagation of eluted phages in *E. Coli* bacteria. To obtain specific phage antibodies that bind to a particular tissue or cell type, the method may be extended by absorbing eluted phage antibodies to remove unwanted specificities. This may be achieved by incubating eluted phage antibodies with sections of other tissues or with sections of the same tissue not containing the target cell of interest and subsequent propagation of unbound phages. In addition, to obtain phage antibodies specific for a particular cell type, for example defined by anatomical localization, individual or groups of cells or pieces of tissue and attached phages may be scraped from the tissue section by a micromanipulator.

The present invention makes available a powerful directed approach for isolating specific antibodies or peptides by antibody or peptide display technologies in combination with solid-phase-dependent peptide synthesis.

The present invention further makes available a powerful directed approach for isolating specific antibodies or peptides by antibody or peptide display technologies in combination with selection procedures on tissue sections or whole tissues.

In one exemplary embodiment of the present invention, the display package is a phage particle which comprises an antibody fusion coat protein that includes the amino acid sequence of an antibody variable region. A library of replicable phage vectors, especially phagemids encoding a library of antibody fusion coat proteins is generated and used to infect suitable host cells. The antibody variable genes used to construct the phage library may be entirely derived from the lymphocytes of individuals or (partly) assembled in vitro as in semi-synthetic phage antibody display libraries. An important determinant of the composition and diversity of these in vitro immune systems is the source of antibody genes used as building blocks to construct the library. Libraries may be assembled from the V regions expressed by the B lymphocytes of an individual known to have mounted a particular immune response as a result of immunization or exposure to an infectious agent. The B cell repertoire in the lymphoid organs of these individuals is enriched for antigen-specific B cells and plasma cells that have undergone clonal expansion and antigen affinity maturation, thus increasing the likelihood of selecting high affinity antibody fragments from the eventual "immunized" phage library. Libraries may be constructed from the V regions expressed by the B cells of "non-immunized" individual in an attempt to recruit all the diversity generated by the natural immune system. Importantly, it should be noted that the B lymphocytes of naive, immunized or infected individuals have been through positive and negative selection forces in primary and secondary lymphoid organs. The net effect of these processes is a B cell repertoire that is less diverse than potentially attainable, based on rearrangement of immunoglobulin gene segments, N region insertions and somatic hypermutation. This in vivo antibody repertoire selection is mirrored in the eventual "naive" phage library. An alternative approach to create diverse libraries exploits the use of large collections of cloned V genes to which randomized CDR3 and JH regions are fused in vitro by PCR. The diversity of these "semi-synthetic" libraries is not constrained by the forces of selec tion acting in the natural immune system. It may be anticipated that such libraries contain specificities not present in the actual antibody repertoires of humans. In the exemplary embodiment a synthetic library is used because it is expected to contain the novel anti-peptide specificities to be selected.

Phage particles formed from the chimaeric protein can be separated by affinity selection based on the ability of the phage-associated antibody to bind to the target peptide synthesized on rods of a pepscan block. The rods on the pepscan block are decorated with individual peptides, the amino acid sequence of which is determined by the amino acid sequence of the target protein. After one or multiple rounds of selection on peptides synthesized on the pepscan block, the phages are selected for binding to the target protein in its native or denatured configuration. For example, the target protein may be a purified recombinant protein expressed in eukaryotic or prokaryotic cells, or a recombinant protein expressed on the cell surface of a prokaryotic or eukaryotic cell. Phages binding to the target protein are eluted, propagated and eventually analyzed for binding.

After one or multipe rounds of affinity selection on peptides, a subtractive or absorption step may be included to obtain phages that discriminate between two closely related peptides. For example, phages that discrimintate between the closely related peptide repre sented by the amino acid sequences DLVYKDPARPKI and DLVYKDPYRPKI may be obtained by affinity selection on the amino acid sequence DLVYKDPARPKI, followed by absorption of phages binding to the amino acid sequence DLVYKDPYRPKI. In the latter step, non-discriminative phages recognizing both peptides are removed and not used for propagation. Antibodies produced by this method can for example be used to distinguish various naturally occuring isoforms of a protein or for immunochemical assays for detecting cell transformations arising due to mutation of an oncogene or an anti-oncogene.

The terms absorption and subtraction refer to the removal of phage antibodies with unwanted specificities from a collection of phages obtained after or prior to affinity selection. The absorption step itself is an affinity selection step on a target expected not to bear the epitopes of interest. The target used for absorption/selection may be tissue sections, cell lines recombinant proteins, peptides etc.

### Examples

The invention now being generally described, it will be more readily understood by reference to the following examples which are included merely for the illustration of certain aspects and embodiments of the present invention and are not intended to limit the invention.

### I. Materials and Methods

Except were indicated otherwise, recombinant DNA methods and microbiological techniques were carried out using standard procedures such as described for example by Sambrook, J. et al. Molecular Cloning: A Laboratory Manual, Cold Spring harbor Laboratory Press, Cold Spring Harbor, NY (1989) and Ausubel, F.M. et al. Current Protocols in Molecular Biology, John Wiley and Sons, Inc. (1995). Specific protocols for propagation and selection of phage antibodies are described in de Kruif, J., et al. Methods in Molecular Biology, (1996?).

### Construction of a synthetic phage antibody display library

The synthetic antibody library was constructed essentially as described (de Kruif et al., J. Mol. Biol. 248, 97 (1995)). Briefly, degenerate oligonucleotides were used to add synthetic CDR3 regions to a collection of 49 previously cloned germline VH genes. Subsequently, these in vitro 'rearranged' VH genes were cloned into a collection of pHEN1 phagemid-derived vectors containing 7 different light chain V regions, fused in frame to the gene encoding the phage minor capsid protein geneIII. Introduction of these constructs into bacteria results, in the presence of helper phage, in the expression of scFv antibody fragments as geneIII fusion proteins on the surface of bacteriophage.

### Preparation of tissue sections

Fixation, freezing, embedding and sectioning of tissues were carried out using standard procedures such as for example described in Zeller, R in Current Protocols in Molecular Biology, pp 14.1.1-14.1.8, Ausubel, F.M. et al. Eds, Green Publishing and Wiley Interscience, New York. Immunohistochemical and immunofluorescent procedures were carried out using standard protoclos such as for example described in Current Protocols in Immunology, pp 5.8.1-5.8.8, Coligan, J.E., et al. Eds. Wiley Interscience, New York.

### Example I: selection using pepscan

### Pepscan synthesis of CD64 peptides

Pepscan synthesis was carried out essentially as described (Geysen, H.M., et al, Proc. Natl. Acad. Sci. USA, 81, 3998 (1984)). In the exemplary embodiment, the extracellular domain of the CD64 molecule was synthesized on 25 polyethylene rods as adjacent, non-overlapping sets of 12-mer peptides, according to the scheme in Table I.
Peptide-specific phages were isolated by affinity binding to the CD64 peptide-covered rods. The rods were blocked by incubation for two hours at room tempertaure by placing the pepscan block for two hours at roomtemperature in wells of a 96-well microtiter plate containing 300 microliter 1x blocking buffer per well (2x blocking buffer: 10% ovalbumin/10% horse serum/1% Tween 80 in PBS). The phages were were mixed 1:1 (v/v) with 2x blocking buffer and incubated for 30 minutes at room temperature. 250 microliter of the blocked phage solution containing approximately 10¹² phages were added per well of a 96 well plate and the 25 rods of the pepscan block were submerged in individual wells and incubated for 3 hours at roomtempera ture. Non-specific phage antibodies were then removed by washing the rods 10 times in PBS/0.5% Tween 80 and one final time in PBS. Phage antibodies bound to the rods were then eluted by adding 300 microliter of 0.1M diethylamine buffer to each well of a microtiter plate and incubation for 5 minutes at roomtemperature. After collection of the 300 microliter, the elution step was repeated. To 600 microliter of eluted phages, 300 microliter of 1M Tris buffer pH 7.4 was added for neutralization.The 900 microliter eluted phage suspension was added to 4 ml 2TY containing 5% glucose and 10 microgram/ml tetracycline. Finally, 5 ml XL-1 blue cells (OD600 of 0.5) were added and the mixture incubated for 30 minutes at 37°C. The cells were centrifuged for 30 minutes at 3000 rpm, the supernatant removed by aspiration and the cells resuspended in 500 microliter of the remaining medium on two TYE plates containg 5% glucose, 100 microgram/ml ampicillin, 10 microgram/ml tetracycline and 5% glucose and the bacteria grown overnight at 37° C. The colonies were scraped from the bacterial plates and used to rescue phage antibody particles as described (de Kruif, J., et al., J. Mol. Biol. 248, 97 (1995)). From the resulting library enriched for peptide-binding phage antibodies, 10¹² phage antibodies in blocking buffer were incubated with the same peptide-covered rods and subjected to subsequent rounds of selection using the same procedure.

### Cleaning of pepscan blocks

Blocks used for ELISA or phage selections were cleaned by submerging the pep scan blocks in disruptbuffer (phosphatebuffer/1%SDS/0.1%beta-mercapto-ethanol, pH 7.2) and placing the block in a ultrasonic waterbath at 70°C for 1 hour.

### Analysis of selected phages in ELISA

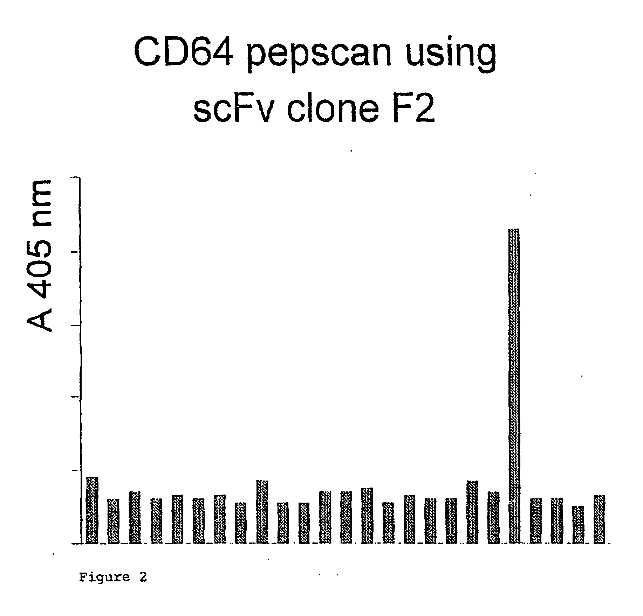

After selection, preparations of monoclonal or polyclonal scFv antibodies were analyzed for the presence of peptide binding in ELISA using the same pepscan blocks used for selections. ScFv preparations instead of phage preparations were used because the latter generated a high background in ELISA. All incubations are carried out in 96-well microtiter plates. The cleaned rods were soaked in 300 microliter PBS for 30 minutes at roomtemperature and pre-coated by incubating each pin for 3 hours at roomtemperature with 150 microliter of blocking buffer (5% ovalbumin/5% horse serum/1% Tween 80, filtered over Whatman 41 paper). The rods were rinsed twice with PBS/0.05% Tween 80 and incubated for 1 hour at roomtemperature with 150 microliter scFv-containing periplasmic preparations prepared as described in de Kruif et al, J. Biol. Chem. 271, 7630 (1996)) mixed with 150 microliter blocking buffer. The rods were washed twice with PBS/0.05% Tween 80 and incubated for 1 hour with 300 microliter culture supernatant of a hybridoma secreting the anti-Myc tag antibody 9E10 (de Kruif, J., et al, J. Biol. Chem. 271, 7630 (1996)). The rods were washed twice with PBS/0.05% Tween 80 and incubated for 1 hour with 300 microliter of rabbit anti-mouse immunoglobulins conjugated to peroxidase, diluted 1:500 in PBS/0.05% Tween 80/1% BSA. Finally, the rods were washed thrice with PBS/0.05% Tween 80 and incubated with ABTS substrate. A representative result of an anti-peptide scFv antibody, affinity-selected after three rounds for binding to peptide # 21 on the CD64 block is shown in figure 2. This scFv antibody specifically bound to peptide #21 used as target for selection but not to other peptide-covered rods in the CD64 pepscan block (figure 2).

### Selection and analysis of pepscan pre-selected phage antibodies by cell sorting.

Phage populations enriched for binding to peptides on the pepscan block may in the second or subsequent rounds be selected for binding to prokaryotic or eukaryotic cells expressing the target protein employing flow cytometry and cell sorting. The procedure for phage selection on intact cells using this procedure has been descibed in detail (de Kruif, J., et al. Proc. Natl. Acad. Sci. USA, 92,3938). Approximately 10¹³ phage antibody particles are blocked for 15 minutes in 4 ml 4% milkpowder in PBS (MPBS). 5 * 10⁶ target cells were added to the blocked phages and the mixture was slowly rotated overnight at 4°C. The following day, cells were washed twice in 50 ml ice-cold PBS/1% BSA. The pelleted cells were resuspended in 2 ml of ice-cold PBS/1% BSA for cell sorting or stained with fluorochrome-labeled monoclonal antibo dies for sorting of a subpopulation of cells. To that end, 25 microliter of fluorochrome-labeled antibodies is added to the pelleted cells and after a 20 minute incubation on ice, cells were washed once with 1% BSA/PBS and resuspended in 2 ml ice-cold PBS/1% BSA. Cell sorting was performed on a FACSvantage.

The procedure for flow cytometric analysis of selected phage antibodies has been described in detail elsewhere (de Kruif, J., et al. Proc. Natl. Acad. Sci. USA, 92,3938). For staining of cells, 100 µl monoclonal phage antibody containing approximately 10¹¹ particles was blocked by adding 50 µl 4% MPBS for 15 minutes at roomtemperature. 5*10⁵ leucocytes in 50 µl PBS/1% BSA were added and incubated on ice for 1 hour. The cells were washed twice in ice-cold PBS/1% BSA. To detect cell-bound phages, the cells were incubated in 10 µl of 1/200 diluted sheep anti-M13 polyclonal antibody (Pharmacia, Uppsala. Sweden), washed twice and incubated in 10 µl of 20 µg/ml PE-labeled donkey anti-sheep polyclonal antibody (Jackson Immu noresearch, West Grove, PA), each for 20 minutes on ice.

### Selection of pepscan pre-selected phage antibodies on whole tissues or tissue sections.

Tissue sections from target organs are prepared and fixed by standard methods. For example, tissues may be submerged in OCT embedding medium and gently frozen by dipping in liquid nitrogen for 1 minute. Using a cryostat, 10 micrometer frozen sections are prepared and collected on microscope slides. The sections are dried for 10 minutes at roomtemperature and fixed in 100% acetone for 5 seconds, followed by drying at roomtemperature. Before incubation with the phage library, the tissue sections are sealed with wax to prevent spreading of fluid and blocked by incubation with MPBS for 30 minutes at roomtemperature. After the blocking step, a total of 10¹² phages from a phage antibody library pre-selected for binding to peptides is blocked with MPBS in a volume of 100 microliter and added to the droplet of MPBS on the slide. The tissue sections are incubated overnight in a moist chamber at 40°C to facilitate binding of phage antibodies.

The sections are rinsed with a total of 2 liter PBS/0.5% Tween 20 for 2 hours at roomtemperature with ten 200 ml changes of PBS/0.5% Tween 20. After rinsing, the phage antibodies are eluted by placing 300 microliter 76 mM citric acid buffer (pH 2.5) on the slide and incubation for 5 minutes at roomtemperature. Add 150 microliter of 1 M Tris-HCL buffer are added to the slide for neutralization and the suspensi on is brought in 3 ml 2 TY medium in a 15 ml tube. 3 ml of XL-1 blue bacteria is added to the tube and infection and production of phages are allowed to proceed as described in the standard protocol. Afer 2-3 rounds of selection, monoclonal phage antibodies are prepared for analysis.

### Analysis of selected phage antibodies by immunohistochemistry

Binding of monoclonal phage antibodies to tissues was determined by immunohistological screening. To this end, 5 µm frozen tissue sections were collected on gelatin coated microscope slides. Sections were then incubated with 50 microliter phage solutions containing 10¹⁰ phage particles in a moist chamber for 30 min. Non-adherent phages were removed by vigorously rinsing the slides in M-PBS-Tw. Bound MoPhabs were visualized by incubation of the sections with a 1:50 diluted sheep anti-M13 peroxidase conjugate, followed by incubation with the substrate diamino benzidene.

### Construction and selection of bi-specific antibodies from collections of phages antibodies affinity selected on pepscan blocks.

Monoclonal phage antibodies selected for binding to the CD64 peptides may be pooled and recloned in display vectors permitting the display of bi-specific antibodies such as for example vectors for diabodies (Holliger, P. et al., Proc. Nat. Acad. Sci. USA, 90, 6444 (1993) or leucine zippered bi-specific antibodies (de Kruif, J., et al. J. Biol. Chem. 271, 7630 (1996).

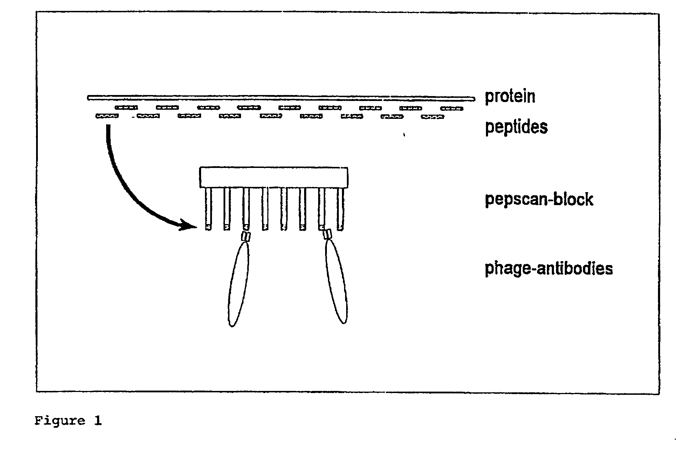

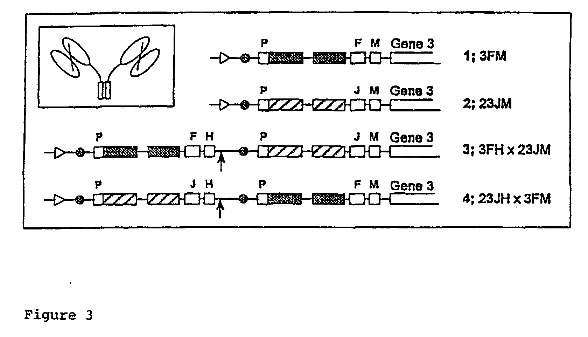

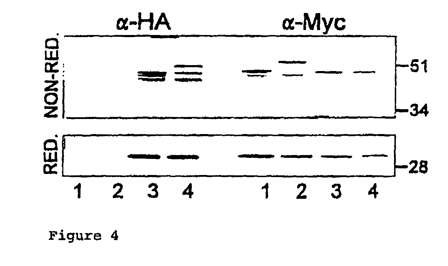

In the exemplary embodiment, a phagemid vector was constructed that facilitates the in vivo bacterial expression and assembly of bispecific (scFv)2 fragments as secreted molecules or g3p fusion proteins on the surface of bacteriophages. The Fos and Jun scFv leucine zipper constructs we described (de Kruif, J., et al. J. Biol. Chem. 271, 7630 (1996); figure 3, 1 and 2) are PCR amplified using primers M13R and PDIM3 (5'-TTT GCA TTC AAG CTT TTA TTA GCC CGC ATA GTC AGG AAC ATC GTA TGG GTA TGC GGC AGC GCA ACC ACC). Primer PDIM3 replaces the Myc tag with a haemagglutinin (HA) tag and adds a stopcodon and a HindIII site to these fragments. PCR products are digested with HindIII and cloned into vector pHEN1 (Hoogenboom, H.R., et al., Nucleic Acid Res. 19, 4133 (1991)) containing a scFv gene fused to the complementary leucine zipper and a Myc tag. In the resulting constructs both scFv-zipper fragments are encoded by a single transcript. Two ribosome binding sites allow the proteins to be translated individually, the first scFv fragment fused to a Fos zipper domain and an HA tag, the second scFv to a Jun zipper and a Myc tag (Figure 3, 3). To determine the effect of the position of the scFv-zippers relative to the g3p protein, an additional construct was made in which the scFv-zipper fragments were reversed (Figure 3, 4). In suppressor E . coli strains, an amber codon inserted between the Myc tag and gene 3 permits the production of phage particles displaying a scFv fragment fused via a zipper region to gene 3. The second scFv will associate with the phage in the periplasmic space by heterodimeri zation of the Fos and Jun leucine zippers, thus creating a phage expressing a bispecific antibody on its surface. In non-suppressor strains, bispecific (scFv)₂ fragments linked by a Fos Jun leucine zipper will be assembled in the periplasmic space.

Expression of (scFv)₂ fragments in non-suppressor bacterial strain SF110-FÂ was monitored by SDS-PAGE followed by blotting to nitrocellulose and detection with Myc tag- and HA tag-specific monoclonal antibodies (MoAbs) (Figure 4, lower panel). In reducing SDS-PAGE, constructs 3 and 4 show expression of HA and Myc tagged proteins of the predicted size. The expression level of Myc tagged scFv fragments obtained with constructs 3 and 4 is lower when compared to that obtained with constructs 1 and 2, perhaps as a result of the simultanuous expression of a second scFv in bacteria harboring the former constructs. In non-reducing SDS-PAGE, only proteins approximately twice the size of a scFv-zipper are visible in all lanes (Figure 4, upper panel). The anti HA-MoAb detects multiple bands, whereas the anti Myc-MoAb detects only one. One of the fragments visualized with the anti-HA MoAb migrates at the same position as the fragments visualized with the anti-Myc MoAb. We hypothesize that this band represents the bispecific antibody and that the other HA-tagged proteins are homodimers resulting from a higher expression level of the first scFv-zipper in these constructs.

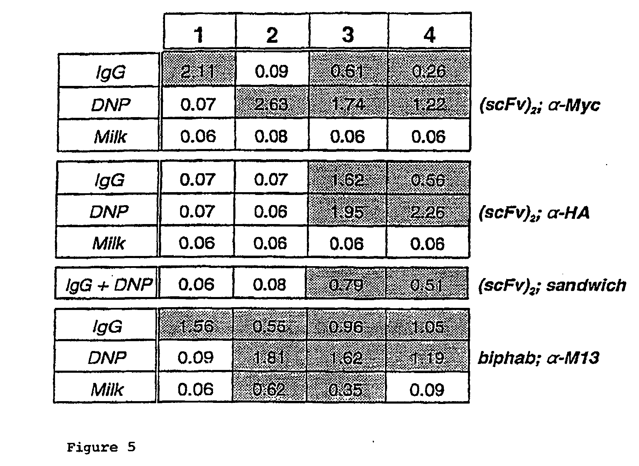

Functionality of the (scFv)₂ fragments was analysed in an ELISA (Figure 5). Using both the Myc tag- and HA tag-specific Moabs for detection, specific binding of the (scFv)₂ fragments to both target antigens could be demonstrated. In a sandwich ELISA we show the (scFv)₂ fragments are truly bispecific in linking two different antigens.
Recombinant phage antibody particles were produced using constructs 1-4 and analysed in an ELISA (Figure 5). Phages from constructs 2 and 3, containing the Jun-zipper fused directly to gene 3 show some non-specific binding to irrelevant antigens. Phages produced using constructs 1 and 4, containing the Fos-zipper fused to gene 3 recognize their target antigens only.

To test the feasability of selecting bispecific phage antibodies from a phage repertoire, bispecific phage particles from construct 4 were spiked in a 1:1000 ratio in an antibody phage display library and subjected to one round of selection in immunotubes coated with either IgG or DNP. The frequency of bispecific phages after selection was estimated by PCR on individual colonies using primers M13R and FOSCON (5'-CGC CAG GAT GAA CTC C, situated in the Fos zipper). Frequencies of bispecific phages after selection were 0/32 (spiked library), 9/32 (selected on IgG) and 6/32 (selected on DNP). Calculated enrichment factors (±250x) are comparable to those obtained using monospecific phage antibodies.

This vector permits the expression of bispecific antibodies that can be functionally expressed on phages using Fos and Jun leucine zippers. It is therefore possible to construct libraries of phages expressing two different scFv fragments and select the desired combinations from these. Furthermore, selected bispecific (scFv)2 fragments can be produced in non-suppressor strains and purified using anti-Myc followed by anti-HA affinity columns.

For the selection of bi-specific antibodies from phage antibody collections pre-selected for binding to peptides on pepscan blocks, care is taken to restrict the number of selection rounds (usually 2-3) on the rod-bound peptides to ensure enrichment for binders but to prevent loss of diversity by overselection. The V gene pools encoding the phage antibodies enriched for binding to the peptides are recloned into the leucine-zipper constructs and bi-specific phage antibodies binding to the target protein are selected by any of the afore-described methods. In addition, it may be envisaged that higher affinity bi-specific antibodies are directly isolated by performing phage selection with the aid of surface palsmon resonance (Jonsson, U., et al. Biotechniques, 11: 620 (1991).

### Example 2: selection on sections

### Preparation of tissue sections

Tissue sections from target organs are prepared and fixed by standard methods. For example, tissues may be embedded in an embedding compound such as OCT and gently frozen by dipping in liquid nitrogen for 1 minute. Using a cryostat, 10 micrometer frozen sections are prepared and collected on microscope slides. The sections are dried for 10 minutes at roomtemperature and fixed in 100% acetone for 5 seconds, followed by drying at roomtemperature.

Alternatively, tissues may be fixed by incubation for several hours in 4% freshly prepared paraformaldehyde solution, dehydrated by serial passage through increasing concentrations of ethanol and embedding in paraffin wax. Paraffin blocks are cut into 6-8 micrometer sections.
Instead of tissue sections, freshly isolated or cultured cells may be used and subjected to the same procedures.

### Affinity selection of phages on tissue sections

The frozen tissue sections are briefly dried at room temperature. The parraffin-embedded tissue sections are placed at 60°C for 1 hour and deparaffinized by immersing in three changes of fresh xylene, 4 minutes each, followed by two changes of 100% ethanol, 2 minutes each. Tissue sections are rehydrated in solutions of decreasing concentrations of ethanol and finally water, and washed with PBS.

Before incubation with the phage library, the tissue sections are sealed with wax to prevent spreading of fluid over the object glass, and blocked by incubation with 1% non-fat milkpowder in phosphate buffered saline (MPBS) for 30 minutes at roomtemperature. After the blocking step, a total of 10¹³ phages from the synthetic phage antibody display library is blocked with MPBS in a volume of 300 microliter and added to the droplet of MPBS on the slide. The tissue sections are incubated over night in a moist chamber at 40°C to facilitate binding of phage antibodies.

The sections are rinsed with a total of 2 liter PBS/0.5% Tween 20 for 2 hours at roomtemperature with 10 200 ml changes of PBS/0.5% Tween 20. After rinsing, the phage antibodies are eluted by placing 300 microliter 76 mM citric acid buffer (pH 2.5) on the slide and incubation for 5 minutes at roomtemperature. 150 microliter of 1 M Tris-HCL buffer is added to the slide for neutrlaization and the suspension is brought in 3 ml 2 TY medium in a 15 ml tube. 3 ml of XL-1 blue bacteria are added to the tube and infection and production of phages are allowed to proceed as described in the standard protocol. Afer 2-3 rounds of selection, monoclonal phage antibodies are prepared for analysis by immunohistochemistry.

### Analysis of selected phage antibodies by immunohistochemistry

Binding of monoclonal phage antibodies to tissues was determined by immunohistological screening. To this end, 5 µm frozen tissue sections were collected on gelatin coated microscope slides. Sections were then incubated with 50 microliter phage solutions containing approximately 10¹⁰ phage particles in a moist chamber for 30 min. Non-adherent phages were removed by vigorously rinsing the slides in MPBS-Tw. Bound phage antibodies were visualized by incubation of the sections with a 1:50 diluted sheep anti-M13 peroxidase conjugate, followed by incubation with diaminobenzidene substrate.

### Absorption of selected phages antibodies for unwanted specificities

A collection of phage antibodies selected for binding to a tissue section may be absorbed for unwanted phage antibody specificities. After elution and neutralization of bound phage antibodies, a 4% (w/v) MPBS solution is added to the eluate to reach a 1% concentration of non-fat milk. The phage solution is subsequently incu bated with a tissue sections of for example another tissue or a different anatomical region of the same tissue. Non-binding phages are removed and propagated directly or incubated with another tissue secion for another round of absorption. This absorption procedure may be repeated multiple times before the eventual non-binding phages are propagated in bacteria.

### Isolation of specific phage antibodies from tissue sections using a micromanipulator.

Individual cells or small regions of tissue and binding phages may be scraped from tissue sections using a micromanipulator or similar device, essentially as described (Kupper, R., et al. EMBO-J, 12 4955 (1993)). This procedure allows the isolation of phages that bind to particular anatomical structure or individual cells in a tissue section. For example, this procedure allows the isolation of endothelial of bloodves sels penetrating a tumor mass or allow the isolation of individual tumor cells. Once scraped from the tissue secion, the bound phages are eluted and propagated as described in the basic protocol.

### Legends to the figures

### Figure 1.

General principle of affinity selection of genetic display packages by binding to peptides synthesized on the rods of a pepscan block.

### Figure 2.

ELISA results of a phage antibody affinity selected for binding to peptide #21 on the CD64 pepscan block. No binding above background to other peptides on the block was observed.

### Figure 3.

Starting material (1 and 2) and bispecific constructs (3 and 4). LacZ promoters (triangles), ribosome binding sites (circles) pelB leaders (P), Pos-zippers (F), Jun-zippers (J), Myc tag (M), HA tag (H) and stopcodons (arrows) are indicated. Closed boxes and hatched boxes represent different scFv specificities.

### Figure 4.

SDS-PAGE / Western blotting analysis. Periplasmic preparations from expressed constructs 1-4 were run under non-reducing (top panel) and reducing (bottom panel) conditions. Proteins were visualized using an HA-tag specific MoAb (left lanes) and a Myc-tag specific MoAb (right lanes) followed by a peroxidase- conjugated antibody.

### Figure 5.

ELISA analysis using construct 1-4 derived antibody fragments. In standard ELISA's, (scFv)₂ fragments were allowed to bind to antigen coated wells and detected using anti-Myc and anti-HA MoAbs followed by a peroxidase-conjugated antibody. Sand wich ELISA's using bispecific (scFv)₂ fragments were performed as described (de Kruif, J et al. J. Biol. Chem. 271, 7630). In phage ELISA, phage antibodies were allowed to bind to antigen coated wells and developed using a peroxidase conjugated anti-M13 antibody. Numbers represent absorbance values. Values three times higher than the background binding to milk-blocked wells are considered to be positive (gray boxes).

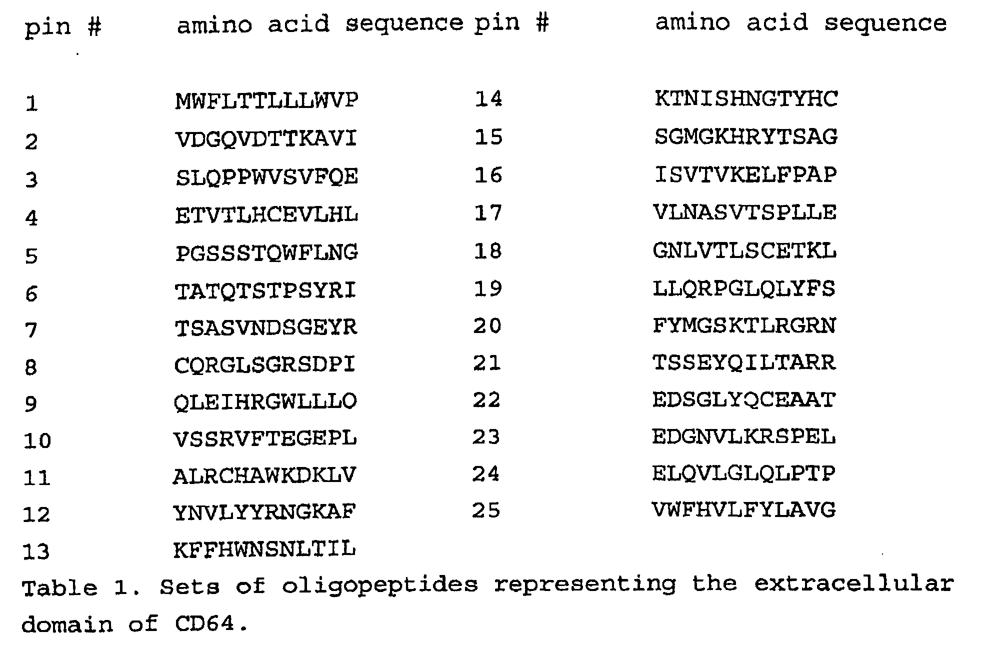

## Claims

1. A method for identifying a peptide capable of specific binding to a proteinaceous target, comprising displaying the peptide on the surface of a replicable display package, synthesizing oligopeptides derived from the proteinaceous target on a solid phase and contacting the specific binding peptide with the oligopeptide to allow for binding.

2. A method for distinguishing between peptides capable of specific binding to a proteinaceous antigen and peptides not having that capability comprising displaying candidate peptides on the surface of a replicable display package, synthesizing oligopeptides derived from the proteinaceous antigen on a solid phase and contacting the candidate peptides with the oligopeptides to allow for binding and washing the solid phase to remove the display packages not specifically bound.

3. A method according to anyone of the aforegoing claims, whereby the replicable display package is a phage particle.

4. A method according to anyone of the aforegoing claims, whereby the replicable display package is a bacterium, a yeast or a spore of a microorganism.

5. A method according to claim 3, whereby the specific binding peptide is displayed on the surface of the phage by insertion of its encoding sequence in a gene encoding a surface protein of said phage.

6. A method according to anyone of the aforegoing claims, whereby the displayed peptide is an immunoglobulin heavy chain, an immunoglobulin light chain, a heavy-light chain pair, a VH, a VL, a Fab, a Fv, an scFv or a di-sulfide-bridged Fv.

7. A method according to anyone of the aforegoing claims whereby the specific binding peptide is a single chain antibody fragment, preferably an scFv.

8. A method according to anyone of the aforegoing claims, further comprising a step whereby the displayed peptides are contacted with a sample not containing the target of interest.

9. A method for screening a library of replicable display packages for peptides capable of specific binding to a prteinaceous target or a fixated biological target comprising subjecting the peptides in the library to a method according to any one of the aforegoing claims.

10. A method according to claim 9 wherein said library is a phage display library.

## Patentansprüche

1. Verfahren zum Identifizieren eines Peptids, das fähig ist, an ein proteinisches Target spezifisch zu binden, wobei das Verfahren umfasst:
Erkennbar Machen des Peptids auf der Oberfläche eines replizierbaren Display-Körpers,
Synthetisieren von Oligopeptiden, die von dem proteinischen Target abgeleitet sind, auf einer festen Phase und
in Kontakt Bringen des spezifisch bindenden Peptids mit dem Oligopeptid, um eine Bindung zu ermöglichen.

2. Verfahren zum Unterscheiden zwischen Peptiden, die fähig sind, an ein proteinisches Antigen spezifisch zu binden und Peptiden, die nicht diese Fähigkeit haben, wobei das Verfahren umfasst:
Erkennbar Machen von in Frage kommenden Peptiden auf der Oberfläche eines replizierbaren Display-Körpers,
Synthetisieren von Oligopeptiden, die von dem proteinischen Antigen abgeleitet sind, auf einer festen Phase und
in Kontakt Bringen der in Frage kommenden Peptide mit den Oligopeptiden, um eine Bindung zu ermöglichen, und
Waschen der festen Phase, um die Display-Körper, die nicht spezifisch gebunden sind, zu entfernen.

3. Verfahren nach einem der vorstehenden Ansprüche, bei dem der replizierbare Display-Körper ein Phagen-Teilchen ist.

4. Verfahren nach einem der vorstehenden Ansprüche, bei dem der replizierbare Display-Körper ein Bakterium, eine Hefe oder eine Spore eines Mikroorganismus ist.

5. Verfahren nach Anspruch 3, bei dem das spezifisch bindende Peptid auf der Oberfläche des Phagens durch Einsetzen seiner codierenden Sequenz in ein Gen, das ein Oberflächenprotein des Phagens codiert, erkennbar gemacht wird.

6. Verfahren nach einem der vorstehenden Ansprüche, bei dem das erkennbar gemachte Peptid eine schwere Immunglobulin-Kette, eine leichte Immunglobulin-Kette, ein Paar aus einer schweren und einer leichten Kette, ein VH, ein VL, ein Fab, ein Fv, ein scFv oder ein Disulfid-verbrücktes Fv ist.

7. Ein Verfahren nach einem der vorstehenden Ansprüche, bei dem das spezifisch bindende Peptid ein Einzelketten-Antikörper-Fragment, bevorzugt ein scFv ist.

8. Verfahren nach einem der vorstehenden Ansprüche, das ferner einen Schritt umfasst, durch den die erkennbar gemachten Peptide mit einer Probe in Kontakt gebracht werden, die das interessierende Target nicht enthalten.

9. Verfahren zum Screenen einer Bibliothek replizierbarer Display-Körper für Peptide, die fähig sind, an ein proteinisches Target oder an ein fixiertes biologisches Target spezifisch zu binden, in dem die Peptide in der Bibliothek einem Verfahren gemäß einem der vorstehenden Ansprüche unterworfen werden.

10. Verfahren nach Anspruch 9, bei dem die Bibliothek eine Phagen-Display-Bibliothek ist.

## Revendications

1. Procédé pour identifier un peptide capable de liaison spécifique à une cible protéique, consistant à afficher le peptide sur la surface d'un paquet d'affichage réplicable, synthétiser des oligopeptides dérivés de la. cible protéique sur une phase solide et mettre en contact le peptide de liaison spécifique avec l'oligopeptide pour permettre la liaison.

2. Procédé pour distinguer entre des peptides capables de liaison spécifique à un antigène protéique et des peptides n'ayant pas cette capacité, consistant à afficher des peptides candidats sur la surface d'un paquet d'affichage réplicable, synthétiser des oligopeptides dérivés de l'antigène protéique sur une phase solide et mettre en contact les peptides candidats avec les oligopeptides pour permettre la liaison et le lavage de la phase solide pour éliminer les paquets d'affichage non liés spécifiquement.

3. Procédé selon l'une quelconque des revendications ci-dessus, dans lequel le paquet d'affichage réplicable est une particule de phage.

4. Procédé selon l'une quelconque des revendications ci-dessus, dans lequel le paquet d'affichage réplicable est une bactérie, une levure ou un spore d'un micro-organisme.

5. Procédé selon la revendication 3, dans lequel le peptide de liaison spécifique est affiché sur la surface du phage par insertion de sa séquence codante dans un gène codant pour une protéine de surface dudit phage.

6. Procédé selon l'une quelconque des revendications précédentes, dans lequel le peptide affiché est une chaîne lourde d'immunoglobulines, une chaîne légère d'immunoglobulines, une paire de chaînes lourde-légère, un VH, un VL, un Fab, un Fv, un scFv ou un Fv à pont disulfure.

7. Procédé selon l'une quelconque des revendications précédentes, dans lequel le peptide de liaison spécifique est un fragment d'anticorps à chaîne unique, de préférence, un scFv.

8. Procédé selon l'une quelconque des revendications précédentes, comprenant de plus une étape dans laquelle les peptides affichés sont mis en contact avec un échantillon ne contenant pas la cible d'intérêt.

9. Procédé pour cribler une banque de paquets d'affichages réplicables pour des peptides capables de liaison spécifique à une cible protéique ou à une cible biologique fixée consistant à soumettre les peptides dans la banque à un procédé selon l'une quelconque des revendications ci-dessus.

10. Procédé selon la revendication 9, dans lequel ladite banque est une banque d'affichage de phages.
